# EUROPEAN PATENT SPECIFICATION

(11) **EP 3 979 255 B1**
(45) Date of publication and mention of the grant of the patent: **10.04.2024**
(21) Application number: 20382872.8
(22) Date of filing: 01.10.2020
(51) Int. Cl.: G16H 20/40, G16H 50/20, A61M 16/00, A61M 16/10, A61B 5/083, A61B 5/00, A61M 16/06, A61B 5/1455

(54) **METHOD AND SYSTEM FOR DETERMINING THE AMOUNT OF OXYGEN REQUIRED BY A USER WITH RESPIRATORY PROBLEMS**
VERFAHREN UND SYSTEM ZUR BESTIMMUNG DER VON EINEM BENUTZER MIT ATMUNGSSTÖRUNGEN BENÖTIGTEN SAUERSTOFFMENGE
PROCÉDÉ ET SYSTÈME PERMETTANT DE DÉTERMINER LA QUANTITÉ D'OXYGÈNE REQUISE PAR UN UTILISATEUR SOUFFRANT DE PROBLÈMES RESPIRATOIRES

(43) Date of publication of application: 06.04.2022
(73) Proprietor: Universitat Politècnica De Catalunya, 08034 Barcelona (ES); Hospital Clínic de Barcelona, 08036 Barcelona (ES); Fundacio de Recerca Clinic Barcelona-Institut d'Investigacions Biomèdiques August Pi I Sunyer, 08036 Barcelona (ES)
(72) Inventor: MASIP BRUIN, Xavier, 08810 Sant Pere de Ribes (ES); PASCUAL SALDAÑA, Heribert, 08729 Castellet i la Gornal (ES); PRIETO GONZÁLEZ, Andrés, 08800 Vilanova i la Geltrú (ES); BLANCO VICH, Isabel, 08029 Barcelona (ES); ALONSO BELTRAN, Albert, 08800 VILANOVA I LA GELTRÚ (ES)
(74) Representative: Segui Quetglas, Margalida

(56) References cited:
- WO-A1-2018/112588
- US-A1- 2006 011 199
- US-A1- 2017 296 104
- SANCHEZ-MORILLO DANIEL ET AL: "Automated Home Oxygen Delivery for Patients with COPD and Respiratory Failure: A New Approach", SENSORS, vol. 20, no. 4, 1178, 20 February 2020 (2020-02-20), XP055783375, DOI: 10.3390/s20041178
- BELTRAME T. ET AL: "Prediction of oxygen uptake dynamics by machine learning analysis of wearable sensors during activities of daily living", SCIENTIFIC REPORTS, vol. 7, 45738, 5 April 2017 (2017-04-05), XP055783444, DOI: 10.1038/srep45738
- FRANÇOIS LELLOUCHE ET AL: "Automated Oxygen Flow Titration to Maintain Constant Oxygenation", RESPIRATORY CARE, vol. 57, no. 8, 17 February 2012 (2012-02-17), pages 1254-1262, XP055281651, US ISSN: 0020-1324, DOI: 10.4187/respcare.01343
- CIRIO S ET AL: "Pilot study of a new device to titrate oxygen flow in hypoxic patients on long-term oxygen therapy", RESPIRATORY CARE, DAEDALUS ENTERPRISES, INC, US, vol. 56, no. 4, 21 January 2011 (2011-01-21), pages 429-434, XP002758915, ISSN: 0020-1324, DOI: 10.4187/RESPCARE.00983 [retrieved on 2011-01-21]
- RICE K. L. ET AL: "AccuO2 Oximetry-Driven Oxygen-Conserving Device Versus Fixed-Dose Oxygen Devices in Stable COPD Patients", RESPIRATORY CARE, vol. 56, no. 12, 31 December 2011 (2011-12-31), pages 1901-1905, XP055783412, US ISSN: 0020-1324, DOI: 10.4187/respcare.01059

## Description

### Technical Field

The present invention relates to a system for determining the amount of oxygen required by a user/patient with respiratory problems.

### Background of the invention

There are known some devices that allow regulating automatically and continuously an amount of oxygen delivered to a patient/user while constantly acquiring physiological parameters of the patients/users, for example the O2 Flow Regulator of the company Dima Italia^{™} or Sanso Via^{™} of the company Sanso Health.

Likewise, OXITONE^{™} provides a SaaS solution focusing on monitoring patients (position, pulse, saturation) and health professionals with the collected data and some statistics through a dashboard.

US2017296104 discloses systems and methods for monitoring pulse oximetry, oxygen debt and oxygen desaturation. US2017296104 is silent about the first data about of plurality of users with respiratory problems being obtained while the plurality of users were performing a cardiorespiratory function test. Further, it does not disclose the step of computing an estimator of the quantity of oxygen to be delivered to the monitored user by implementing at least one second artificial intelligence algorithm on the customized user's behavioral model, nor the first, second and third periods of time being specified and being configurable.

WO2018112588 discloses a system and a method based on machine learning to modulate oxygen levels and performing adjustments in real time to the volume and concentration of mixtures of O₂, CO₂ and N₂ that are administered for inspiration. Compared to the present invention, it does not disclose the user's behavioral model characterizing user behavior in terms of saturation vs effort, and adjusting of the behavioural model in an iterative way as suggested by the present invention.

### Summary of the invention

The scope of the invention is defined by the appended claims 1-15.

### Description of the Invention

An object of the present invention is to provide a highly efficient, usable and secure solution aimed at monitoring and caring for patients/users with chronic respiratory diseases, technically supported by a novel patients' behavior modelling solution through an Artificial Intelligence (Al) engine and a well-defined caring methodology covering the whole disease lifecycle, i.e., assisting the initial diagnose steps and providing a mechanism to adapt oxygen delivery either alone or associated with ventilatory support devices (BIPAP, CPAP, etc.), intended to support the needs for real-time oxygen supply in said patients/users. Indeed, the present invention focusses on significantly improving patients' quality of life by increasing their tolerance to the activities of daily living and avoiding sedentary behaviors.

A method for determining the amount of oxygen required by a user/ patient with respiratory problems is disclosed. This method does not make part of the scope of invention. In this not-claimed method, first data about a plurality of users with respiratory problems is stored, particularly anonymized, in a memory or database, the first data including oxygen saturation and heart beat frequency parameters of the users which were acquired while the plurality of users were performing a cardiorespiratory function test, for example the 6 minute walking test (6MWT).

The method comprises a) collecting, via a wearable data acquisition device, second data from a monitored user with respiratory problems while the monitored user is performing the cardiorespiratory function test at a first location, for example a hospital or a health center premises; b) computing, via a processing unit, a user's behavioral model that characterizes user behavior in terms of saturation vs effort by implementing one or more (first) artificial intelligence algorithms on the first and second data; c) collecting, via the wearable data acquisition device, every certain first configurable period of time t1, third data of the monitored user while the monitored user is performing an activity at a second location, for example the user's home or outdoors; d) adjusting, by a processing unit, every certain second configurable period of time t2, the computed user's behavioral model by means of implementing the one or more (first) artificial intelligence algorithms on the third data, providing a customized user's behavioral model as a result; and e) computing, via a processing unit, an estimator of the quantity of oxygen to be delivered to the monitored user by implementing at least one (second) artificial intelligence algorithm on the customized user's behavioral model.

The second and third data also include oxygen saturation and heart beat frequency parameters. In some embodiments, the first, second and third data can also include geolocation information, among other information/parameters.

Particularly, the method is (continuously) repeated in order to train the customized user's behavioral model with updated data coming from user's activity monitoring (i.e. coming from the collected third data). Therefore, steps c) - e) are repeated every certain third configurable period of time, for example every day, every two days, every week, etc.

The artificial intelligence algorithms considered by the present invention are dynamic, intended to easing the adoption of novel Al solutions and removing those showing low accuracy. In an embodiment, both the first and second artificial intelligence algorithms are the same. In this case, present invention considers at least the following three algorithms: SVM, XGBOOST and deep learning to train the data and generate distinct user's behavioral models. It should be noted that in some embodiments only one of the above algorithms needs to be implemented. In another embodiment, the second artificial intelligence algorithm is different to the first artificial intelligence algorithm, for example, in this case the second artificial intelligence algorithm can comprise a deep learning approach built upon a neural network.

The plurality of users can be separated into groups in the database according to different parameters such as disease, gender and age, among others. The plurality of users share similar individual and disease traits with the monitored user.

The first configurable period of time t1 can be comprised in a range between 1 to 5 minutes and the second configurable period of time t2 is once per day.

The activity may comprise walking via a known or unknown route or a daily activity at the monitored user's home.

The method may also display the customized user's behavioral model and/or the computed estimator on a screen of a computing device.

The method may also comprise sending an audible and/or visual warning signal to the monitored user and/or to a healthcare staff if the computed estimator is different than a threshold. In addition, the method can also update the estimator based on the cardiorespiratory function test to deliver basal oxygen.

The method can also comprise applying the one or more artificial intelligence algorithms to the first, second and/or third data, issuing N datasets as a result, each dataset turning into a different trained model representing patient's behavior. Moreover, the method particularly can also comprise selecting the optimal model of said N different models through a competition and verification process.

Present invention provides a system for determining the amount of oxygen required by a user/patient with respiratory problems. The system includes a memory or database, a wearable data acquisition device adapted to be placed on a monitored user having respiratory problems, and one or more processing units operatively associated with or connected to the wearable data acquisition device.

The memory or database is adapted to store first data about a plurality of users with respiratory problems, the first data including oxygen saturation and heart beat frequency parameters of the plurality of users which were acquired while the plurality of users were performing a cardiorespiratory function test, for example the 6 minute walking test (6MWT).

In the proposed system the one or more processing units are configured to: compute a user's behavioral model by implementing at least one (first) artificial intelligence algorithm on the first data and on second data, the second data being collected from one or more sensors in the wearable data acquisition device while the monitored user is performing the cardiorespiratory function test at a first location, the second data including oxygen saturation and heart beat frequency parameters; provide a customized user's behavioral model by adjusting, every certain second configurable period of time t2, the computed user's behavioral model by means of implementing the at least one (first) artificial intelligence algorithm on third data, the third data being collected, every certain first configurable period of time t1, from the one or more sensors while the monitored user is performing an activity at a second location, the third data including oxygen saturation and heart beat frequency parameters; compute an estimator of the quantity of oxygen to be delivered to the monitored user by implementing at least one (second) artificial intelligence algorithm on the customized user's behavioral model. The steps of providing the customized user's behavioral model and computing the estimator of the quantity of oxygen to be delivered to the monitored user, are repeated every certain third configurable period of time, such that the customized user's behavioral model is repeatedly trained with updated data.

In some embodiments, the first and second artificial intelligence algorithms are the same and comprise a SVM algorithm, a XGBOOST algorithm and/or a deep learning algorithm. Alternatively, in other embodiments, the first and second artificial intelligence algorithms are different. In this latter case the second artificial intelligence algorithm can comprise a deep learning approach built upon a neural network.

The one or more processing units can be included either in a smartphone or in a cloud computing server. In some embodiments, one of the processing units is included in a cloud computing server which is configured to perform the computing of the user's behavioral model, and one of the processing units is included in a smartphone or a computing device (such as an ad-hoc device) which is configured to perform the computing of the estimator.

In an embodiment, the one or more sensors comprise an oxygen saturation level sensor and a heat rate sensor. The one or more sensor can also include a GPS or geolocation sensor.

In an embodiment, the wearable data acquisition device further includes an oxygen delivery element such as a cylinder valve, among others.

In an embodiment, the wearable data acquisition device comprises a wrist-mounted or a head-mounted device.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Thus, the present invention can provide:
- Integrated solution including two well defined phases, planning (modelling patient's behavior) and runtime (estimating real-time patients' oxygen support needs).
- Agnostic to the oxygen system provider.
- Supporting external data sources for improved decision making.
- Heart beat and saturation monitoring.
- Al Engine estimating patients' performance modeling and oxygen support needs.
- Sampling period customized and adaptable.
- 2 dashboards, Frontend for health professionals (F4H) and Frontend for Live (F4L) for health professionals and patients respectively, both connected through a Backend repository.
- Data sensitive care (privacy concerns).
- Distributed smartness and data analytics at cloud.
- Routes training protocol for exercise pattern recommendation.
- Adherence support.
- Warnings to both the patient and the health professionals' team.
- Diagnose recommendation to the health professionals' team.
- Real-time patient monitoring.
- Real-time oxygen adaptation to the needs of the patient.
- Direct connectivity between all involved actors (patient, caregivers, professionals).
- Warnings to the oxygen provider about real status (capacity, etc.) of its device.
- Micro-services oriented architecture.

### Brief Description of the Drawings

The previous and other advantages and features will be more fully understood from the following detailed description of embodiments, with reference to the attached figures, which must be considered in an illustrative and non-limiting manner, in which:
Fig. 1 schematically illustrates the blocks architecture of the present invention, according to an embodiment.
Fig. 2 schematically illustrates the planning phase, according to an embodiment.
Fig. 3 graphically illustrates an example of how the behavioral model is downloaded in the runtime phase.
Fig. 4 graphically illustrates an example of how the behavioral model is queried and the estimator acquired in the runtime phase.

### Detailed Description of the Invention

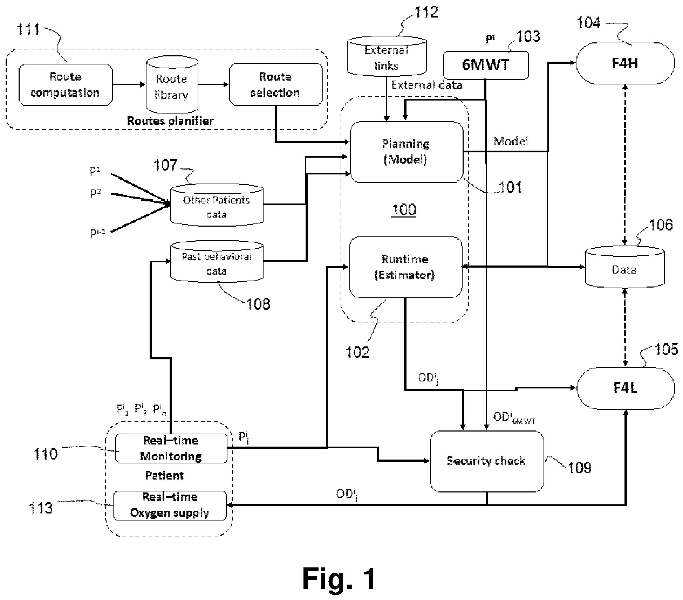

Fig. 1 shows an embodiment of the proposed architecture of the invention representing a two-phase design supported by the Al engine 100. All additional features and functionalities as defined in the present invention are inferred from these two-phase design, including adherence, warnings, communication, routes training, etc.

According to this embodiment, a planning phase 101 is fed by data collected from the 6 minute walking test (6MWT) 103, which is collected at hospital premises in a highly controlled scenario. In some cases, in particular when the individual patients/users 1 only run the 6MWT once per month, or even less, during the planning phase 101 data replication for individual users will not be considered. In fact, according to the current protocol, health professionals estimate the oxygen dose to be delivered to the patients after usually running only once the 6MWT, according to a fixed table mapping oxygen saturation into oxygen delivery. A runtime phase 102 will collect users' data from their daily activities (walking, etc.), thus putting together a large set of individualized information for each individual user 1. This information is used by the planning phase 101 in next iterations.

With regard to the Al Engine 100, each phase 101, 102 include a block deploying specific Al-based solutions. At the start, the input for the planning phase 101 for each individual user 1 will be the overall set of data collected (a maximum can be set) for patients/users with similar characteristics (disease, age, gender, etc.), i.e. the first data as termed in the claims, along with results of the individual user 6MWT, i.e. the second data as termed in the claims. In further iterations of the proposed method, past user's activity data (i.e. third data) will be also used by the planning phase 101. In this way a behavioral model is computed for each individual user 1. Indeed, the output of the planning phase 101 is an individual user's behavioral model, to be daily trained with updated data coming from daily user's activity monitoring (i.e. collected by the real time monitoring module 110 that is sensing/monitoring the user 1). In an embodiment, the process of updating/adjusting the behavioral model is done daily, usually when there is no user activity and all data for that day is collected (normally at nights).

Consequently, the planning phase outcome is a user's behavioral model modeling patient's saturation vs the expected effort, which as days goes on, is continuously trained and updated and so becoming much more accurate.

All collected and processed information is stored in a backend repository and made accessible to health professionals and users through two customized dashboards 104, 105, the F4H and F4L respectively. The two dashboards 104, 105 facilitate users and professionals to get connected to the system on each phase and to easily get the required information.

When the user 1 performs an activity at home or outdoors, such as walking, the runtime phase 102 starts. In this phase 102 the individual user 1 is continuously monitored in previously defined periods of time, for instance every 1-5 minutes, in particular 1 minute, and the collected values are used to query the computed behavioral model turning into an estimator of the oxygen needs for the next period, i.e., the next minute. The data collected in this phase 102 via the cited real time monitoring module 110 updates (particularly in a daily basis) previous data used in the planning block 101 and that is stored in the database 108, thus adjusting the behavioral model accuracy and thus increasing the chances for the runtime block 102 to deliver an oxygen prediction much closer to real user's needs. The outcome of the Al Engine 100, i.e. the behavioral model and the oxygen estimator, will be better as larger the information collected from the user 1 is. Hence, in some embodiments, to prevent deviations, mainly in the first weeks, a security check 109 is introduced intended to correct sudden large changes in the oxygen estimation. In this case, when a previously threshold is passed, the basal oxygen computed according to the 6MWT, is recommended to the user 1.

Finally, the output of the security check 109 goes to the oxygen-supply module 113 to deliver/provide the estimated amount/quantity of oxygen to the user 1.

As also indicated in Fig. 1, the present invention also facilitates the inclusion of a set of categorized routes (Routes planner 111) to support the exercise recommendation to be given by the health professional to the user 1. To that end, the present invention, in an embodiment, delivers advice at the planning phase 101 and updates the recommendation while monitoring and self-learning about the user behavior.

The smart decision process proposed by the present invention can be distributed throughout cloud and local facilities, and is supported by the cited Al Engine 100, consisting in the Al-assisted strategy technically providing the desired objectives on each phase 101, 102. It must be emphasized that both phases 101, 102 are active, each in different time windows. Indeed, the planning phase 101 remains active when the user 1 is performing no activity (in other words, is not producing data), training the behavioral model. Differently, the runtime phase 102 is active when the user 1 activity is monitored, collecting data sent to the database and querying the computed model to deliver the proper oxygen estimation. To this end, the user 1 should wear a wearable data acquisition device and a software APP running in a mobile device 10 (e.g. a Smartphone) should be open.

### Planning Phase.-

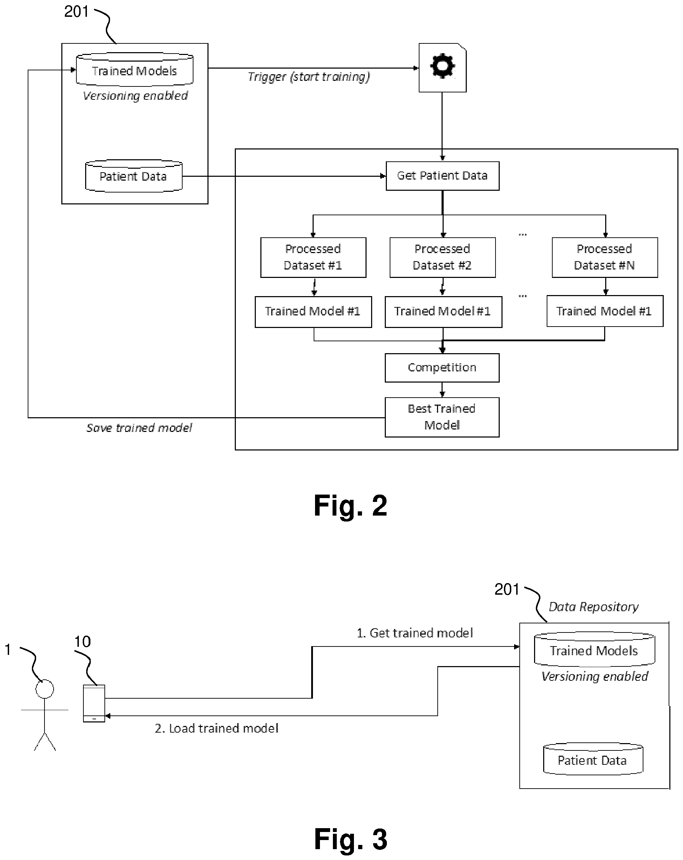

During the planning phase 101, the Al Engine 100 elaborates the behavioral model characterizing users oxygen support needs vs the effort to be done. To this end, in the initial iterations, the data used to train the model (at least including heart rate and SPO2, and optionally geolocation data), comes from the set of similar users, which are particularly separated into different groups according to the disease, gender, age, etc., along with the individual user data obtained from the 6MWT. Then, in an embodiment, as shown in Fig. 2, the whole set of data can be split into N datasets through several artificial intelligence strategies, each training the data and generating a model according to several parameters. Finally, the N different models compete to check for accuracy, finally selecting the optimal one. The model selected is stored in a database 201 waiting to be queried during the runtime phase 102. This process can be handled using different artificial intelligence algorithms such as SVM, XGBOOST and/or Deep Learning. The rationale for using these algorithms is to balance different data training options, e.g., SVM is a supervised strategy suitable to classify data, XGBOOST supports large amount of data with multiple variables and Deep Learning is an unsupervised neural network strategy able to manage big data processes. The computational required by each artificial intelligence strategy is also different, hence the Al Engine 100 will also consider that variable to select the set of artificial intelligence strategies to be used, mainly when reaching out to very large amount of data.

As stated before, particularly, the system proposes to run the planning phase 101 at nights when no user activity is reported (unless night activity is to be monitored as well). Indeed, the overall set of collected data is tentatively adjusted to 24 hours, thus putting together data enough to run a new training process, so each training iteration in the planning phase 101 will comprise data collected in one day.

In an embodiment, the planning phase 101 is run/executed in a cloud computing environment; not limitative as in other embodiments it can be run/executed by a computer device such as a mobile phone 10, among others.

### Runtime Phase.-

The runtime phase 102 starts when both the user 1 wears the wearable device (to be monitored) and when the software APP is open. Basically, in this phase 102, the user's behavioral model computed in the planning phase 101 is queried with the real-time user collected data to obtain a prediction of the oxygen required by the user 1 in the next time window.

In an embodiment, since queries to the behavioral model are triggered every minute, the prediction time window is also for 1 minute, meaning to say, that every minute the present invention computes and delivers an estimator of the real oxygen support need for next minute.

In a particular embodiment, the Al strategy to use in the runtime phase 102 is the one used to build the behavioral model delivered by the planning phase 101, i.e., the strategy computing the behavioral model selected after the competition process out of the N produced models during the planning phase 101. Alternatively, the Al strategy in this runtime phase 102 can be based in a recurrent neural network. In addition, as this is a real-time process, particularly all computation actions included in this phase 102 are done close to the user 1, i.e., in a device physically deployed at the edge, either an ad-hoc system or the own user's mobile phone 10.

The Runtime phase 102 is split into two actions, start-up and prediction:
Start-up: This action is responsible for obtaining the last updated behavioral model trained with the last collected data (see Fig. 3). It consists on a request from the user's mobile phone 10 (or an ad-hoc device deployed as part of the present invention) to the database 201 where all trained behavioral models (i.e. the customized behavioral models) are stored. Consequently, the database 201 will return the last updated behavioral model. This action must guarantee that the user's mobile phone 10 (or an ad-hoc device) contains the last trained, or customized, behavioral model. Once the customized behavioral model is uploaded into the edge device and after some checks (queries to check response time, wrong values due for example to wrong collected data, etc.) next action starts. If checks are not successfully passed, an old version (day before) of the customized behavioral model will be used.

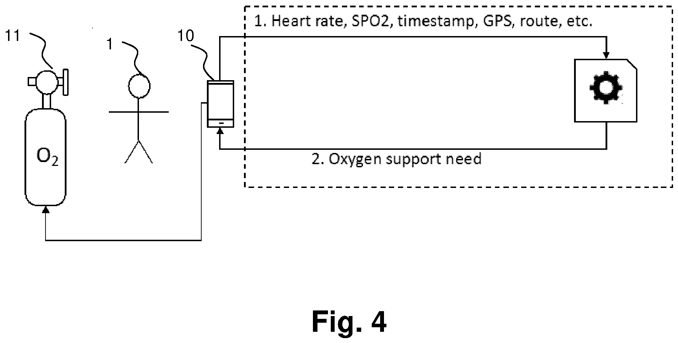

Prediction: This action is responsible for issuing the query to the behavioral model, including some values (e.g., Heart Rate, SPO2, and optionally GPS, Timestamp, route characteristics, etc.) and computing an estimator of the oxygen support need for the next time interval, e.g. next minute. This action will remain active while the software APP is on, see Fig. 4.

The whole set of data can be accessed through the two proposed frontends 104, 105, connected through the backend, also facilitating the direct communication between users, health professionals and caregivers.

The present invention can consider different potential incremental deployment options, as will be detailed next. However, beyond the conditions defined on each scenario, new approaches can be defined depending on the potential links and agreements to be defined with oxygen providers and pulse oximeters manufacturers.

In an embodiment, the software APP is deployed. The APP comprises two clear physical wireless interfaces, one to the pulse oximeter responsible for collecting the data from the user 1 and the other one to an external valve (provided by an oxygen cylinder provider). The APP will collect the data, process it according to the algorithmic strategy already defined and will forward the decision on the oxygen to be delivered to an external valve controller. Although this is the lightest version, it includes all functionalities as envisioned for the present invention.

In an embodiment, the cited APP is extended, termed FOOXY suite, by adding specific hardware, thus having the need to use the mobile device 10 of the user 1. The FOOXY suite interfaces the pulse oximeter 11 (see Fig. 4) through a wireless connection and connects the valve to a wired connection. Actually, the FOOXY Suite hardware may be deployed along with the cylinder. The complete FOOXY suite can include the electronic and the valve required to handle the oxygen dose. The present invention only requires a wireless connection to the pulse oximeter 11.

In some embodiments, the wearable data acquisition device (not shown in the figures) is a light bracelet responsible for data monitoring and collection. Moreover, the invention includes a box to be attached to the oxygen cylinder valve, responsible for the data processing and connectivity.

According to a particular embodiment, the present invention builds upon a database located at cloud premises storing all information collected from patients/users, particularly under severe anonymized guarantees, to feed and train the Al engine 100. When a new user 1 starts using the invention the two phases 101, 102 start. The planning phase 101 can start by collecting some personal user information, including age, disease, gender. This information is used to register the user 1, thus facilitating the user 1 access to the F4L dashboard 105, where the user 1 can visualize all data and statistics. Once done, the user 1 runs the 6MWT and the invention starts collecting data (the second data). Considering these individual data and also the data stored in the database 107 for similar users, the present invention delivers the first time, the following outcomes:
- A basal oxygen delivery dose, that can be considered by the health professional in a disease diagnose.
- A behavioral model characterizing the user oxygen needs vs effort done. This behavioral model is continuously (e.g. daily) trained with updated acquired activity data of the user 1, so its inaccuracy lowers as time goes on.
- Additionally, an exercise pattern defined as a set of routes (from the routes library) can be also proposed to the user 1 by the health professional, so preventing morbidity problems.

Then, every time the user 1 uses the present invention, the proposed system will monitor its behavior by at least collecting saturation, heartbeat, and optionally, localization parameters, will store the collected data in the database 108 to train the behavioral model and will recommend the oxygen support value when queried by the software APP in the runtime phase 102. A warning signal can be provided to the user 1 and/or to the health professionals or caregivers if any potential issue happens during the runtime monitoring.

A check process is included to guarantee that the Al Engine 100 is performing as expected. To that end, the computed estimator is updated when the difference between the real-time monitoring and the estimator value goes far beyond a threshold, to be defined by the health professional. While this strategy guarantees that higher deviations (error in the estimator) are corrected, it also makes not necessary for the system to be continuously monitoring the status of the user 1. In an embodiment, the checking policy advises for doing this process at the very beginning when the data used to train the Al is not individual, thus expecting higher deviations. However, as the data is updated with patient's individual data, such checking can be shifted in time.

At any time, the user 1 can look at the F4L information about his/her status, obtain statistics, and also use it to interact with other users. Similarly, the health professionals may use the F4P to see users' status and also to get any warning and message coming from the users. This data processing will be very useful to accommodate further treatments and to analyze users' evolution and reaction to current efforts.

According to the present invention, different activities can be also considered, including:
- Tracking of the user's activity: Includes predefined routes, exercise videos, feedback on the activity carried out, etc.
- Activity diary: Journal containing the schedule of planned activities.
- Communication: Deployment of communication in different environments, including doctor-user, user-user, user-caregiver, etc.
- Educational: alimentation, prevention, good habits, medication intake control and reminders, general information on events, publications and studies, associations/foundation involvement.
- Family support: The user's relatives have a strong link with the disease and their environment despite not suffering the disease. The present invention must facilitate the connection and communication of the family as well as the customization of a fluid and simple communication and collaborative environment.
- Alerts: Definition of the type of warnings to include in the system to facilitate both control by the medical team and self-control and monitoring by the user himself and his environment.

The present invention can also give support and provide strategies to deal with some of the problems that the medical community has not yet solved, therefore including the following as basic functionalities:
- Better management of physical activity as part of the non-pharmacological treatment, in the global strategy of patient management.
- Customized and precise supply of individualized oxygen volume to each user in response to real needs within the medical parameters defined by the medical chart.
- Remote monitoring and real-time visualization of the user's condition and its evolution based on the data obtained, the alerts displayed and the behavioral curves to be defined.
- Objectivize the results obtained in the testing phase using the 6MWT to validate the degree and potential impact of the different hypoxemic diseases.
- Share "best practices" through a collaborative medical platform to improve management strategies for these patients.
- Allow a better and more accurate diagnosis on the potential impact of each disease on each user specifically on the possible development of daily physical activities.
- Facilitate the effort made to eliminate or reduce sedentary lifestyle, being considered a high risk factor in health for the entire population, through the generation that facilitates monitoring and adherence of users to physical activity.
- Interoperability, based on facilitating better coordination of medical personnel, by connecting users, specialists and caregivers.
- Data processing for the generation of data analytics that facilitate decision making for medical personnel, as well as any other derivatives that may be estimated in the future.

Various aspects of the proposed method, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with image processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

A machine-readable medium may take many forms, including but not limited to, a tangible storage medium, a carrier wave medium or physical transmission medium. Non-volatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described herein may be embodied in a hardware device, it may also be implemented as a software only solution—e.g., an installation on an existing server. In addition, image processing as disclosed herein may be implemented as a firmware, firmware/software combination, firmware/hardware combination, or a hardware/firmware/software combination. The present disclosure and/or some other examples have been described in the above. According to descriptions above, various alterations may be achieved. The topic of the present disclosure may be achieved in various forms and embodiments, and the present disclosure may be further used in a variety of application programs.

The scope of the present invention is defined in the following set of claims.

## Claims

1. A system for determining the amount of oxygen required by a user with respiratory problems, comprising:
a memory or database configured to store first data about a plurality of users with respiratory problems, the first data at least including oxygen saturation and heart beat frequency parameters of the plurality of users which were acquired while the plurality of users were performing a cardiorespiratory function test;
a wearable data acquisition device configured to be placed on a monitored user having respiratory problems; and
one or more processing units operatively associated with the wearable data acquisition device, the one or more processing units being configured to:
a) compute a user's behavioral model that characterizes user behavior in terms of user oxygen support needs vs an effort to be done by implementing at least one first artificial intelligence algorithm on the first data and second data, the second data being collected from one or more sensors in the wearable data acquisition device while the monitored user is performing the cardiorespiratory function test at a first location, the second data at least including oxygen saturation and heart beat frequency parameters;
b) provide a customized user's behavioral model by adjusting, every certain second configurable period of time t2, the computed user's behavioral model by means of implementing the at least one first artificial intelligence algorithm on third data, the third data being collected, every certain first configurable period of time t1, from the one or more sensors while the monitored user is performing an activity at a second location, the third data at least including oxygen saturation and heart beat frequency parameters;
c) compute an estimator of the quantity of oxygen to be delivered to the monitored user by implementing at least one second artificial intelligence algorithm on the customized user's behavioral model,
wherein steps b)-c) are repeated every certain third configurable period of time, such that the customized user's behavioral model is repeatedly trained with updated data.

2. The system of claim 1, wherein the at least one first artificial intelligence algorithm comprises a SVM algorithm, a XGBOOST algorithm and/or a deep learning algorithm.

3. The system of claim 1, wherein the first and second artificial intelligence algorithms comprise a SVM algorithm, a XGBOOST algorithm or a deep learning algorithm.

4. The system of claim 1 or 2, wherein the second artificial intelligence algorithm comprises a deep learning approach built upon a neural network.

5. The system of any one of the previous claims, wherein the plurality of users are separated into groups in the database according to different parameters including at least: disease, gender and age, and wherein the plurality of users share similar individual and disease traits with the monitored user.

6. The system of any one of the previous claims, wherein the first configurable period of time t1 is comprised in a range between 1 to 5 minutes and the second configurable period of time t2 is once per day.

7. The system of any one of the previous claims, wherein the cardiorespiratory function test comprises the 6 minute walking test.

8. The system of any one of the previous claims, wherein the activity comprises walking via a known or unknown route or a daily activity at the monitored user's home.

9. The system of any one of the previous claims, further comprising a computing device, wherein the one or more processing units are further configured to display the customized user's behavioral model and/or the computed estimator on a screen of said computing device.

10. The system of claim 9, wherein if the computed estimator is different to a threshold the one or more processing units are further configured to send an audible and/or visual warning signal to the monitored user and/or to a healthcare staff and to update the computed estimator based on the cardiorespiratory function test to deliver a basal oxygen.

11. The system of any one of the previous claims, further comprising a smartphone (10), wherein the one or more processing units are included in the smartphone (10).

12. The system of any one of the previous claims 1-10, further comprising a cloud computing server, wherein the one or more processing units are included in the cloud computing server.

13. The system of any one of the previous claims 1-10, wherein at least one of the one or more processing units is included in a cloud computing server which is configured to perform the computing of the user's behavioral model, and wherein at least one of the one or more processing units is included in a smartphone (10) or a computing device which is configured to compute the estimator.

14. The system of any one of the previous claims, wherein the one or more sensors comprise at least an oxygen saturation level sensor and a heat rate sensor, wherein the wearable data acquisition device further includes an oxygen delivery element, and wherein the wearable data acquisition device comprises a wrist-mounted or a head-mounted device.

15. A computer program product comprising instructions which, when the program is executed by a computer, cause the computer to carry out a method for determining the amount of oxygen required by a user with respiratory problems, by:
a) computing a user's behavioral model that characterizes user behavior in terms of user oxygen support needs vs an effort to be done by implementing at least one first artificial intelligence algorithm on first data about a plurality of users with respiratory problems and on second data,
the first data at least including oxygen saturation and heart beat frequency parameters of the users which were acquired while the plurality of users were performing a cardiorespiratory function test, and
the second data being collected from one or more sensors in a wearable data acquisition device placed on a monitored user having respiratory problems while the monitored user is performing the cardiorespiratory function test at a first location, the second data at least including oxygen saturation and heart beat frequency parameters;
b) providing a customized user's behavioral model by adjusting, every certain second configurable period of time t2, the computed user's behavioral model by means of implementing the at least one first artificial intelligence algorithm on third data, the third data being collected, every certain first configurable period of time t1, from the one or more sensors while the monitored user is performing an activity at a second location, the third data at least including oxygen saturation and heart beat frequency parameters;
c) computing an estimator of the quantity of oxygen to be delivered to the monitored user by implementing at least one second artificial intelligence algorithm on the customized user's behavioral model,
wherein steps b)-c) are repeated every certain third configurable period of time, such that the customized user's behavioral model is repeatedly trained with updated data.

## Patentansprüche

1. System zum Bestimmen der von einem Benutzer mit Atmungsstörungen benötigten Sauerstoffmenge, Folgendes umfassend:
einen Speicher oder eine Datenbank, der/die ausgebildet ist, um erste Daten über eine Vielzahl von Benutzern mit Atmungsstörungen zu speichern, wobei die ersten Daten mindestens Sauerstoffsättigungs- und Herzschlagfrequenzparameter der Vielzahl von Benutzern einschließen, die erfasst wurden, während die Vielzahl von Benutzern einen kardiorespiratorischen Funktionstest durchführten;
eine tragbare Datenerfassungsvorrichtung, die ausgebildet ist, um an einem überwachten Benutzer platziert zu werden, der Atmungsstörungen aufweist; und
eine oder mehrere Verarbeitungsvorrichtungen, die operativ mit der tragbaren Datenerfassungsvorrichtung assoziiert sind, wobei die eine oder mehreren Verarbeitungsvorrichtungen für Folgendes ausgebildet sind:
a) Berechnen eines Verhaltensmodells des Benutzers, das das Verhalten des Benutzers hinsichtlich des Sauerstoffunterstützungsbedarfs des Benutzers gegenüber einer durchzuführenden Anstrengung charakterisiert, indem mindestens ein erster Künstliche-Intelligenz-Algorithmus auf den ersten Daten und den zweiten Daten implementiert wird, wobei die zweiten Daten von einem oder mehreren Sensoren in der tragbaren Datenerfassungsvorrichtung gesammelt werden, während der überwachte Benutzer den kardiorespiratorischen Funktionstest an einem ersten Ort durchführt, wobei die zweiten Daten mindestens Sauerstoffsättigungs- und Herzschlagfrequenzparameter einschließen;
b) Bereitstellen eines maßgeschneiderten Verhaltensmodells des Benutzers durch Einstellen des berechneten Verhaltensmodells des Benutzers in jedem gewissen zweiten konfigurierbaren Zeitraum t2 mittels Implementieren des mindestens einen ersten Künstliche-Intelligenz-Algorithmus auf dritte Daten, wobei die dritten Daten in jedem gewissen ersten konfigurierbaren Zeitraum t1 von dem einen oder den mehreren Sensoren gesammelt werden, während der überwachte Benutzer eine Aktivität an einem zweiten Ort durchführt, wobei die dritten Daten mindestens Sauerstoffsättigungs- und Herzschlagfrequenzparameter einschließen;
c) Berechnen eines Schätzers der Menge an Sauerstoff, die an den überwachten Benutzer abzugeben ist, durch Implementieren mindestens eines zweiten Künstliche-Intelligenz-Algorithmus auf dem maßgeschneiderten Verhaltensmodell des Benutzers,
wobei die Schritte b)-c) in jedem gewissen dritten konfigurierbaren Zeitraum wiederholt werden, so dass das maßgeschneiderte Verhaltensmodell des Benutzers wiederholt mit aktualisierten Daten trainiert wird.

2. System nach Anspruch 1, wobei der mindestens eine erste Künstliche-Intelligenz-Algorithmus einen SVM-Algorithmus, einen XGBOOST-Algorithmus und/oder einen Deep-Learning-Algorithmus umfasst.

3. System nach Anspruch 1, wobei der erste und der zweite Künstliche-Intelligenz-Algorithmus einen SVM-Algorithmus, einen XGBOOST-Algorithmus oder einen Deep-Learning-Algorithmus umfassen.

4. System nach Anspruch 1 oder 2, wobei der zweite Künstliche-Intelligenz-Algorithmus einen Deep-Learning-Ansatz umfasst, der auf einem neuronalen Netz aufbaut.

5. System nach einem der vorhergehenden Ansprüche, wobei die Vielzahl der Benutzer in Gruppen in der Datenbank nach mindestens folgenden verschiedenen Parametern unterschieden wird, die mindestens Folgendes einschließen: Krankheit, Geschlecht und Alter, und wobei die Vielzahl der Benutzer ähnliche Individual- und Krankheitsmerkmale mit dem überwachten Benutzer teilen.

6. System nach einem der vorhergehenden Ansprüche, wobei der erste konfigurierbare Zeitraum t1 einen Bereich von 1 bis 5 Minuten umfasst ist und der zweite konfigurierbare Zeitraum t2 einmal pro Tag ist.

7. System nach einem der vorhergehenden Ansprüche, wobei der kardiorespiratorische Funktionstest den 6-Minuten-Gehtest umfasst.

8. System nach einem der vorhergehenden Ansprüche, wobei die Aktivität das Gehen auf einer bekannten oder unbekannten Route oder eine tägliche Aktivität am Wohnort des überwachten Benutzers umfasst.

9. System nach einem der vorhergehenden Ansprüche, ferner umfassend eine Rechenvorrichtung, wobei die eine oder mehrere Verarbeitungseinheiten ferner ausgebildet sind, um das maßgeschneiderte Verhaltensmodell des Benutzers und/oder den berechneten Schätzer auf einem Bildschirm der Rechenvorrichtung anzuzeigen.

10. System nach Anspruch 9, wobei, wenn sich der berechnete Schätzer von einem Schwellenwert unterscheidet, die eine oder die mehreren Verarbeitungseinheiten ferner ausgebildet sind, um ein akustisches und/oder visuelles Warnsignal an den überwachten Benutzer und/oder an das Gesundheitspersonal zu senden und den berechneten Schätzer basierend auf dem kardiorespiratorischen Funktionstest zu aktualisieren, um einen basalen Sauerstoff abzugeben.

11. System nach einem der vorhergehenden Ansprüche, ferner umfassend ein Smartphone (10), wobei die eine oder mehrere Verarbeitungseinheiten in dem Smartphone (10) eingeschlossen sind.

12. System nach einem der vorhergehenden Ansprüche 1-10, ferner umfassend einen Cloud-Computing-Server, wobei die eine oder mehrere Verarbeitungseinheiten in dem Cloud-Computing-Server eingeschlossen sind.

13. System nach einem der vorhergehenden Ansprüche 1-10, wobei mindestens eine der einen oder mehreren Verarbeitungseinheiten in einem Cloud-Computing-Server eingeschlossen ist, der ausgebildet ist, um die Berechnung des Verhaltensmodells des Benutzers durchzuführen, und wobei mindestens eine der einen oder mehreren Verarbeitungseinheiten in einem Smartphone (10) oder einer Rechenvorrichtung eingeschlossen ist, die ausgebildet ist, den Schätzer zu berechnen.

14. System nach einem der vorhergehenden Ansprüche, wobei der eine oder die mehreren Sensoren mindestens einen Sauerstoffsättigungspegelsensor und einen Herzschlagfrequenzparameter umfassen, wobei die tragbare Datenerfassungsvorrichtung ferner ein Sauerstoffabgabeelement einschließt, und wobei die tragbare Datenerfassungsvorrichtung eine am Handgelenk montierte oder eine am Kopf montierte Vorrichtung umfasst.

15. Computerprogrammprodukt, das Anweisungen umfasst, die, wenn das Programm von einem Rechner ausgeführt wird, den Rechner veranlassen, ein Verfahren zum Bestimmen der von einem Benutzer mit Atmungsstörungen benötigten Sauerstoffmenge durchzuführen, durch:
a) Berechnen eines Verhaltensmodells des Benutzers, das das Verhalten des Benutzers hinsichtlich des Sauerstoffunterstützungsbedarfs des Benutzers gegenüber einer durchzuführenden Anstrengung charakterisiert, indem mindestens ein erster Künstliche-Intelligenz-Algorithmus auf ersten Daten über eine Vielzahl von Benutzern mit Atmungsstörungen und auf zweiten Daten implementiert wird,
wobei die ersten Daten mindestens Sauerstoffsättigungs- und Herzschlagfrequenzparameter der Vielzahl von Benutzern einschließen, die erfasst wurden, während die Vielzahl von Benutzern einen kardiorespiratorischen Funktionstest durchführten; und
wobei die zweiten Daten von einem oder mehreren Sensoren in einer tragbaren Datenerfassungsvorrichtung gesammelt werden, die an einem überwachten Benutzer platziert wird, der Atmungsstörungen aufweist, während der überwachte Benutzer den kardiorespiratorischen Funktionstest an einem ersten Ort durchführt, wobei die zweiten Daten mindestens Sauerstoffsättigungs- und Herzschlagfrequenzparameter einschließen;
b) Bereitstellen eines maßgeschneiderten Verhaltensmodells des Benutzers durch Einstellen des berechneten Verhaltensmodells des Benutzers in jedem gewissen zweiten konfigurierbaren Zeitraum t2 mittels Implementieren des mindestens einen ersten Künstliche-Intelligenz-Algorithmus auf dritte Daten, wobei die dritten Daten in jedem gewissen ersten konfigurierbaren Zeitraum t1 von dem einen oder den mehreren Sensoren gesammelt werden, während der überwachte Benutzer eine Aktivität an einem zweiten Ort durchführt, wobei die dritten Daten mindestens Sauerstoffsättigungs- und Herzschlagfrequenzparameter einschließen;
c) Berechnen eines Schätzers der Menge an Sauerstoff, die an den überwachten Benutzer abzugeben ist, durch Implementieren mindestens eines zweiten Künstliche-Intelligenz-Algorithmus auf dem maßgeschneiderten Verhaltensmodell des Benutzers,
wobei die Schritte b)-c) in jedem gewissen dritten konfigurierbaren Zeitraum wiederholt werden, so dass das maßgeschneiderte Verhaltensmodell des Benutzers wiederholt mit aktualisierten Daten trainiert wird.

## Revendications

1. Système permettant de déterminer la quantité d'oxygène requise par un utilisateur souffrant de problèmes respiratoires :
une mémoire ou une base de données configurée pour stocker des premières données relatives à une pluralité d'utilisateurs souffrant de problèmes respiratoires, les premières données comprenant au moins des paramètres de saturation en oxygène et de fréquence des battements cardiaques de la pluralité d'utilisateurs qui ont été acquis pendant que la pluralité d'utilisateurs effectuaient un test de la fonction cardiorespiratoire ;
un dispositif vestimentaire d'acquisition de données configuré pour être placé sur un utilisateur surveillé souffrant de problèmes respiratoires ; et
une ou plusieurs unités de traitement associées de manière opérationnelle au dispositif vestimentaire d'acquisition de données, l'une ou plusieurs unités de traitement étant configurées pour :
a) calculer un modèle comportemental de l'utilisateur qui caractérise le comportement de l'utilisateur en termes de besoins d'apport d'oxygène par rapport à un effort à réaliser en mettant en oeuvre au moins un premier algorithme d'intelligence artificielle sur les premières données et des deuxièmes données, les deuxièmes données étant collectées à partir d'un ou plusieurs capteurs dans le dispositif vestimentaire d'acquisition de données pendant que l'utilisateur surveillé effectue le test de la fonction cardiorespiratoire à un premier emplacement, les deuxièmes données comprenant au moins des paramètres de saturation en oxygène et de fréquence des battements cardiaques ;
b) fournir un modèle comportemental de l'utilisateur personnalisé en ajustant, à chaque deuxième période de temps configurable t2 donnée, le modèle comportemental calculé de l'utilisateur au moyen de la mise en oeuvre de l'au moins un premier algorithme d'intelligence artificielle sur des troisièmes données, les troisièmes données étant collectées, à chaque première période de temps configurable t1 donnée, à partir de l'un ou plusieurs capteurs pendant que l'utilisateur surveillé effectue une activité à un deuxième emplacement, les troisièmes données comprenant au moins des paramètres de saturation en oxygène et de fréquence des battements cardiaques ;
c) calculer un estimateur de la quantité d'oxygène à fournir à l'utilisateur surveillé en mettant en oeuvre au moins un deuxième algorithme d'intelligence artificielle sur le modèle comportemental de l'utilisateur personnalisé,
dans lequel les étapes b)-c) sont répétées à chaque troisième période de temps configurable donnée, de sorte que le modèle comportemental de l'utilisateur personnalisé est entraîné de manière répétée avec des données actualisées.

2. Système selon la revendication 1, dans lequel l'au moins un premier algorithme d'intelligence artificielle comprend un algorithme SVM, un algorithme XGBOOST et/ou un algorithme d'apprentissage profond.

3. Système selon la revendication 1, dans lequel les premier et deuxième algorithme d'intelligence artificielle comprennent un algorithme SVM, un algorithme XGBOOST ou un algorithme d'apprentissage profond.

4. Système selon la revendication 1 ou 2, dans lequel le deuxième algorithme d'intelligence artificielle comprend une approche d'apprentissage profond basée sur un réseau neuronal.

5. Système selon l'une quelconque des revendications précédentes, dans lequel la pluralité d'utilisateurs sont séparés en groupes dans la base de données en fonction de différents paramètres, dont au moins : la maladie, le sexe et l'âge, et dans lequel la pluralité d'utilisateurs partage des traits individuels et pathologiques similaires à ceux de l'utilisateur surveillé.

6. Système selon l'une quelconque des revendications précédentes, dans lequel la première période de temps configurable t1 est comprise dans une plage d'entre 1 et 5 minutes et la deuxième période de temps configurable t2 est d'une fois par jour.

7. Système selon l'une quelconque des revendications précédentes, dans lequel le test de la fonction cardiorespiratoire comprend le test de marche de 6 minutes.

8. Système selon l'une quelconque des revendications précédentes, dans lequel l'activité comprend la marche sur un itinéraire connu ou inconnu ou une activité quotidienne au domicile de l'utilisateur surveillé.

9. Système selon l'une quelconque des revendications précédentes, comprenant en outre un dispositif informatique, dans lequel l'une ou plusieurs unités de traitement sont configurées en outre pour afficher le modèle comportemental de l'utilisateur personnalisé et/ou l'estimateur calculé sur un écran dudit dispositif informatique.

10. Système selon la revendication 9, dans lequel si l'estimateur calculé est différent d'un seuil, l'une ou plusieurs unités de traitement sont configurées en outre pour envoyer un signal d'avertissement sonore et/ou visuel à l'utilisateur surveillé et/ou à un personnel soignant et pour mettre à jour l'estimateur calculé sur la base du test de la fonction cardiorespiratoire afin d'administrer un oxygène de base.

11. Système selon l'une quelconque des revendications précédentes, comprenant en outre un smartphone (10), dans lequel l'une ou plusieurs unités de traitement sont incluses dans le smartphone (10).

12. Système selon l'une quelconque des revendications précédentes 1-10, comprenant en outre un serveur informatique en nuage, dans lequel l'une ou plusieurs unités de traitement sont incluses dans le serveur informatique en nuage.

13. Système selon l'une quelconque des revendications précédentes 1-10, dans lequel au moins une de l'une ou plusieurs unités de traitement est incluse dans un serveur informatique en nuage qui est configuré pour effectuer le calcul du modèle comportemental de l'utilisateur, et dans lequel au moins une de l'une ou plusieurs unités de traitement est incluse dans un smartphone (10) ou un dispositif informatique qui est configuré pour calculer l'estimateur.

14. Système selon l'une quelconque des revendications précédentes, dans lequel l'un ou plusieurs capteurs comprennent au moins un capteur de niveau de saturation en oxygène et un capteur de fréquence cardiaque, dans lequel le dispositif vestimentaire d'acquisition de données comprend en outre un élément d'administration d'oxygène, et dans lequel le dispositif vestimentaire d'acquisition de données comprend un dispositif monté sur le poignet ou monté sur la tête.

15. Produit de programme informatique comprenant des instructions qui, lorsque le programme est exécuté par un ordinateur, amènent l'ordinateur à mettre en oeuvre une méthode pour déterminer la quantité d'oxygène requise par un utilisateur souffrant de problèmes respiratoires, par :
a) le calcul d'un modèle comportemental de l'utilisateur qui caractérise le comportement de l'utilisateur en termes de besoins d'apport d'oxygène par rapport à un effort à réaliser en mettant en oeuvre au moins un premier algorithme d'intelligence artificielle sur des premières données concernant une pluralité d'utilisateurs souffrant de problèmes respiratoires et sur des deuxièmes données,
les premières données comprenant au moins des paramètres de saturation en oxygène et de fréquence des battements cardiaques des utilisateurs qui ont été acquis pendant que la pluralité d'utilisateurs effectuait un test de la fonction cardiorespiratoire, et
les deuxièmes données étant collectées à partir d'un ou plusieurs capteurs dans un dispositif vestimentaire d'acquisition de données placé sur un utilisateur surveillé souffrant de problèmes respiratoires pendant que l'utilisateur surveillé effectue le test de la fonction cardiorespiratoire à un premier emplacement, les deuxièmes données comprenant au moins des paramètres de saturation en oxygène et de fréquence des battements cardiaques ;
b) la fourni d'un modèle comportemental de l'utilisateur personnalisé en ajustant, à chaque deuxième période de temps configurable t2 donnée, le modèle comportemental calculé de l'utilisateur au moyen de la mise en oeuvre de l'au moins un premier algorithme d'intelligence artificielle sur des troisième données, les troisièmes données étant collectées, à chaque première période de temps configurable t1 donnée, à partir de l'un ou plusieurs capteurs pendant que l'utilisateur surveillé effectue une activité à un deuxième emplacement, les troisième données comprenant au moins des paramètres de saturation en oxygène et de fréquence des battements cardiaques ;
c) le calcul d'un estimateur de la quantité d'oxygène à fournir à l'utilisateur surveillé en mettant en oeuvre au moins un deuxième algorithme d'intelligence artificielle sur le modèle comportemental de l'utilisateur personnalisé,
dans lequel les étapes b)-c) sont répétées à chaque troisième période de temps configurable donnée, de sorte que le modèle comportemental de l'utilisateur personnalisé est entraîné de manière répétée avec des données actualisées.
